# EUROPEAN PATENT SPECIFICATION

(11) **EP 2 007 677 B1**
(45) Date of publication and mention of the grant of the patent: **21.11.2012**
(21) Application number: 07712507.8
(22) Date of filing: 12.03.2007
(51) Int. Cl.: C01B 33/14, C01B 33/18, C01B 37/00, C01B 39/08, C07D 301/12, B01J 29/04

(54) **SILICON-TITANIUM MIXED OXIDE-CONTAINING DISPERSION FOR THE PRODUCTION OF TITANIUM-CONTAINING ZEOLITES**
SILICIUM-TITAN-MISCHOXID ENTHALTENDE DISPERSION ZUR HERSTELLUNG VON TITANHALTIGEN ZEOLITHEN
DISPERSION CONTENANT UN MELANGE D'OXYDES DE SILICIUM ET DE TITANE UTILISEE POUR PRODUIRE DES ZEOLITES CONTENANT DU TITANE

(30) Priority: 15.04.2006 DE 102006017700
(43) Date of publication of application: 31.12.2008
(73) Proprietor: Evonik Degussa GmbH, 45128 Essen (DE)
(72) Inventor: LORTZ, Wolfgang, 63607 Wächtersbach (DE); SCHUMACHER, Kai, 65719 Hofheim (DE)
(86) International application number: PCT/EP2007/052278
(87) International publication number: WO 2007/118738

(56) References cited:
- EP-A1- 0 814 058
- US-A1- 2003 129 153

## Description

The invention relates to a process for the production of a silicon-titanium mixed oxide powder-containing dispersion for the production of titanium-containing zeolites.

From EP-A-814058, the use of silicon-titanium mixed oxide powders for the production of titanium-containing zeolites is known. Titanium-containing zeolites are efficient catalysts for the oxidation of olefins with hydrogen peroxide. They are obtained by hydrothermal synthesis starting from silicon-titanium mixed oxide powders in the presence of a template. In EP-A-814058, it is disclosed that pyrogenic silicon-titanium mixed oxides with a silicon dioxide content of 75 and 99.9 wt.-% can be used for this. Particularly advantageous is a composition which contains from 90 to 99.5 wt.-% silicon dioxide and 0.5 to 5 wt.% titanium dioxide. As templates, amines, ammonium compounds or alkali (alkaline earth) metal hydroxides can be used.

A disadvantage of the process disclosed in EP-A-814058 is that it leads to products whose catalytic activity is often not reproducible and is often not adequate.

The patent application US2003/129153 discloses silicon-titanium mixed oxide powders prepared by flame hydrolysis, which may contain traces of impurities up to 0.5 wt.%, however generally not more than 100 ppm; a ratio by weight of SiO₂/TiO₂ from 0.01 to 99 % including specific values such as 97 wt.%; powders having BET surface areas from 10 to 300 m²/g, including specific values such as 250 m²/g, these values obtained by means of the process parameters. This document also discloses the use of those powders for the preparation of dispersions.

The purpose of the present invention was therefore to provide a silicon-titanium mixed oxide in a form which makes it possible to produce titanium-containing zeolites of high catalytic activity.

The object of the invention is a process for the production of a dispersion containing pyrogenic silicon-titanium mixed oxide powders with a silicon dioxide content of 75 to 99.99 wt.% and a titanium dioxide content of 0.01 to 25 wt.%, water and a basic, quaternary ammonium compound, wherein the mean aggregate diameter of the particles of the silicon-titanium mixed oxide powder in the dispersion is 200 nm at most, comprising the steps:
- water, which, in the event that silicon-titanium mixed oxide powder incorporated later leads to a pH value of the aqueous phase of <2 or >4, is adjusted to pH values of 2 to 4 by addition of acids or bases, is circulated from a holding tank through a rotor/stator machine, and
- via a filling device, silicon-titanium mixed oxide powder is introduced continuously or discontinuously into the shear zone between the grooves of the rotor teeth and the stator grooves, with the rotor/stator machine running, in a quantity such that a predispersion with a solids content of 20 to 40 wt.-% results, and
- after all the silicon-titanium mixed oxide powder has been added, the filling device is closed, and shearing continued such that the shear rate lies in the range between 10000 to 40000 sec⁻¹, and
- then, while maintaining the dispersion conditions, a basic, quaternary ammonium compound is added, and optionally water, before the addition of the ammonium compound.

It was found that on use of this dispersion, which contains particles of this fineness, the reaction time which is needed for the production of titanium-containing zeolites is markedly reduced. Preferably, the mean aggregate diameter is less than 100 nm.

Pyrogenic should be understood to refer to metal mixed oxide particles obtained by flame oxidation and/or flame hydrolysis. During this, oxidisable and/or hydrolysable starting materials are oxidised or hydrolysed as a rule in a hydrogen-oxygen flame. The metal mixed oxide particles are as pore-free as possible and have free hydroxyl groups on the surface. They are present in the form of aggregated primary particles.

The BET surface area of the pyrogenic silicon-titanium mixed oxide powders is not limited. It has however been found advantageous if the BET surface area lies in a range from 20 to 400 m²/g and in particular from 50 to 300 m²/g. The use of a silicon-titanium mixed oxide powder of high BET surface area in combination with a small mean aggregate diameter in the dispersion is particularly advantageous for the production of titanium-containing zeolites.

It has further been found advantageous if the dispersion contains a pyrogenic silicon-titanium mixed oxide powder, wherein the proportions of Na, K, Fe, Co, Ni, Al, Ca and Zn are each less than 50 ppm, in particular less than 25 ppm. Such a dispersion leads to titanium-containing zeolites with high catalytic activity.

The dispersion also contains a basic, quaternary ammonium compound. These are preferably tetraalkylammonium hydroxides such as for example tetraethylammonium hydroxide, tetra-n-propylammonium hydroxide and/or tetra-n-butylammonium hydroxide. Basic, quaternary ammonium compounds serve as templates which determine the crystal structure through incorporation into the crystal lattice. Tetra-n-propylammonium hydroxide is preferably used for the production of titanium silicalite-1 (MFI structure), tetra-n-butylammonium hydroxide for the production of titanium silicalite-2 (MEL structure) and tetraethylammonium hydroxide for the production of titanium β-zeolites (BEA crystal structure).

The ratio water / silicon-titanium mixed oxide powder is preferably 10 ≤ mol water/ mol silicon-titanium mixed oxide ≤ 20. The range 12 ≤ mol water/ mol silicon-titanium mixed oxide ≤ 17 is particularly preferred.

The content of quaternary, basic ammonium compound in the dispersion is not limited. If the dispersion is to be stored for a prolonged period, it can be advantageous to add to it only a part of the quantity of the dispersion necessary for the production of a titanium-containing zeolite. Preferably, the quaternary, basic ammonium compound can be added in a quantity such that a pH value of 9 to 11, in particular 9.5 to 10.5, results. In this pH range, the dispersion displays good stability.

If for example the dispersion is to be used directly after its production for the production of a titanium-containing zeolite, the dispersion can already contain the whole quantity of quaternary, basic ammonium compound. Preferably then 0.12 ≤ mol ammonium compound / mol silicon-titanium mixed oxide < 0.20, and 0.13 ≤ mol ammonium compound / mol silicon-titanium mixed oxide ≤ 0.17 is particularly preferred.

For the production of a titanium-containing zeolite, the dispersion, optionally with further addition of the basic, quaternary ammonium compound, is processed at a temperature of 150 to 220°C for a period of less than 12 hours. The crystals obtained are separated by filtration, centrifugation or decantation and washed with a suitable washing liquid, preferably water.

The crystals are then dried as required, and calcined at a temperature between 400°C and 1000°C, preferably between 500°C and 750°C, in order to remove the template.

A titanium-containing zeolite, may be obtainable by this process.

The titanium-containing zeolite is obtained in powder form. For its use as an oxidation catalyst, as required it is converted into a form suitable for the use, e.g. into micropellets, spheres, tablets, solid cylinders, hollow cylinders or honeycombs by known methods for the shaping of catalysts in powder form, such as for example pelleting, spray-drying, spray pelleting or extrusion.

The titanium-containing zeolite can be used as a catalyst in oxidation reactions with hydrogen peroxide. In particular, it can be used as a catalyst in the epoxidation of olefins by means of aqueous hydrogen peroxide in a solvent miscible with water.

### Examples

### Example 1: Production of a silicon-titanium mixed oxide powder

5.15 kg/hr of silicon tetrachloride and 0.15 kg/hr of titanium tetrachloride are vaporised. The vapours are transferred into a mixing chamber by means of 15 Nm³/hr of nitrogen as carrier gas. Separately from this, 2 Nm³/hr of hydrogen and 8 Nm³/hr of primary air are introduced into the mixing chamber. In a central pipe, the reaction mixture is fed into a burner and ignited. Here the flame burns in a water-cooled flame tube. In addition, 15 Nm³/hr of secondary air is introduced into the reaction space. The powder produced is separated in a filter connected downstream and then treated with a counter-current of hydrogen at 520°C.

The powder displays the following values:
Silicon dioxide 96.6 wt.%
Titanium dioxide 3.4 wt.%
BET surface area 80 m2/g

### Example 2: Production of a Dispersion (according to invention)

32.5 kg of deionised water are first placed in a 100 I stainless steel batch vessel. Next, 17.5 kg of the silicon-titanium mixed oxide powder from Example 1 are inducted under shear conditions using the suction tube of the Ystral Conti-TDS 4 (Stator groove: 6 mm ring and 1 mm ring, rotor/stator gap ca. 1 mm). After completion of the induction, the inlet nozzle is closed and the 35 weight percent predispersion is sheared for a further 10 mins at 3000 rpm. Undesired warming of the dispersion due to the high energy input is counteracted with a heat exchanger and the temperature rise limited to max. 40 °C. Owing to the acidic nature of the pyrogenically produced silicon-titanium mixed oxide powder, the pH value of the dispersion is ca. 3.6.

Next, 28.6 kg of deionised water are added, and a pH value of 10.0 is rapidly established under intensive shearing and thorough mixing with 1.0 kg of tetra-n-propylammonium hydroxide solution (40 wt.-% in water).

The dispersion displays the following values:
Water/silicon-titanium mixed oxide 11.7
Mean aggregate diameter 94 nm (determined on Horiba LA 910)

### Example 3: Production of a Dispersion (Comparison)

1 g of tetra-n-propylammonium hydroxide solution (40 wt.-% in water) are added to 17.5 g of the silicon-titanium mixed oxide powder from Example 1 in 61.1 ml of water under dispersing conditions by means of a dissolver, and dispersed for 30 mins. The dispersion obtained has a markedly higher viscosity in comparison to Example 3. Coarse aggregates can clearly be discerned as well as finer ones.

The dispersion displays the following values:
Water/silicon-titanium mixed oxide 13.2
Tetrapropylammonium hydroxide/silicon-titanium mixed oxide 0.14
Mean aggregate diameter 256 nm

### Example 4: Production of a titanium-containing zeolite

505 g of the dispersion from Example 2 are first placed in a polyethylene beaker, 46.7 g of deionised water and 130.6 g of a tetra-n-propylammonium hydroxide solution (40 wt.-% in water) are added and firstly aged for four hours at 80°C with stirring and then crystallised for 10 hours at 180°C in an autoclave. The solid obtained is separated from the mother liquor by centrifugation, washed with 3 x 250 ml portions of deionised water, dried at 90°C and calcined in an atmosphere of air for four hours at 550°C.

The Xray diffraction diagram of the crystals obtained from Example 4 shows the diffraction pattern typical for the MFI structure, and the IR spectrum the characteristic bands at 960 cm⁻¹. The UV/visible light spectrum shows that the sample is free from titanium dioxide and titanates.

Example 5 is performed analogously to Example 4, but with the use of the dispersion from Example 3.

In contrast to Example 4, Example 5 yields markedly more coarse zeolite aggregates. In the catalytic epoxidation of propylene, the product from Example 4 displays a higher activity than that from Example 5.

## Claims

1. Process for the production of a dispersion containing pyrogenic silicon-titanium mixed oxide powder with a silicon dioxide content of 75 to 99.99 wt.% and a titanium dioxide content of 0.01 to 25 wt.-%, water and a basic, quaternary ammonium compound, the particles of the silicon-titanium mixed oxide powders in the dispersion having a mean aggregate diameter of 200 nm at most,
comprising the steps:
- water, which, in the event that the silicon-titanium mixed oxide powder incorporated later leads to a pH value of the aqueous phase of <2 or >4, is adjusted to pH values of 2 to 4 by addition of acids or bases, is circulated from a holding tank through a rotor/ stator machine, and
- via a filling device, silicon-titanium mixed oxide powder is continuously or discontinuously introduced into the shear zone between the grooves of the rotor teeth and the stator groove, with the rotor/stator machine running, in a quantity such that a predispersion with a solids content of 20 to 40 wt.% results, and
- after all the silicon-titanium mixed oxide powder has been added, the filling device is closed, and shearing continued such that the shear rate lies in the range between 10000 to 40000 sec⁻¹, and
- next, while maintaining the dispersing conditions, a basic, quaternary ammonium compound and optionally water, before the addition of the ammonium compound, are added.

## Patentansprüche

1. Verfahren zur Herstellung einer Dispersion, enthaltend pyrogenes Silicium-Titan-Mischoxidpulver mit einem Anteil an Siliciumdioxid von 75 bis 99,99 Gew.-% und einem Anteil an Titandioxid von 0,01 bis 25 Gew.-%, Wasser und eine basische, quaternäre Ammoniumverbindung, wobei der mittlere Aggregatdurchmesser der Partikel des Silicium-Titan-Mischoxidpulvers in der Dispersion maximal 200 nm beträgt,
umfassend die Schritte:
- Wasser, welches, falls das später eingebrachte Silicium-Titan-Mischoxidpulver zu einem pH-Wert der wässrigen Phase von <2 oder >4 führt, durch Zugabe von Säuren oder Basen auf pH-Werte von 2 bis 4 eingestellt ist, wird aus einer Vorlage über eine Rotor-/Statormaschine im Kreis geführt, und
- über eine Einfüllvorrichtung wird kontinuierlich oder diskontinuierlich und bei laufender Rotor-/Stator-maschine eine solche Menge Silicium-Titan-Mischoxidpulver in die Scherzone zwischen den Schlitzen der Rotorzähne und dem Statorschlitz eingebracht, dass eine Vordispersion mit einem Feststoffgehalt von 20 bis 40 Gew.-% resultiert, und
- man schließt, nachdem alles Silicium-Titan-Mischoxidpulver zugegeben ist, die Einfüllvorrichtung und schert so weiter, dass die Scherrate im Bereich zwischen 10000 bis 40000 s⁻¹ liegt, und
- man gibt anschließend unter Beibehalten der Dispergierbedingungen eine basische, quaternäre Ammoniumverbindung und optional, vor der Zugabe der Ammoniumverbindung, Wasser hinzu.

## Revendications

1. Procédé pour la production d'une dispersion contenant une poudre pyrogène d'oxyde mixte de silicium-titane avec une teneur en dioxyde de silicium de 75 à 99,99 % en poids et une teneur en dioxyde de titane de 0,01 à 25 % en poids, de l'eau et un composé d'ammonium quaternaire basique, les particules de la poudre d'oxyde mixte de silicium-titane dans la dispersion ayant un diamètre moyen d'agrégat de 200 nm au maximum, comprenant les étapes suivantes :
- de l'eau, qui dans l'éventualité où la poudre d'oxyde mixte de silicium-titane incorporée ultérieurement conduirait à une valeur de pH de la phase aqueuse < 2 ou > 4 est ajustée à des valeurs de pH de 2 à 4 par addition d'acides ou de bases, est mise en circulation à partir d'un réservoir de stockage à travers une machine à rotor/stator, et
- par le biais d'un dispositif de remplissage, une poudre d'oxyde mixte de silicium-titane est introduite de façon continue ou discontinue dans la zone de cisaillement entre les sillons des dents du rotor et le sillon du stator, avec la machine à rotor/stator en fonctionnement, dans une quantité telle qu'une prédispersion avec une teneur en matières solides de 20 à 40 % en poids en résulte, et
- après que toute la poudre d'oxyde mixte de silicium-titane a été ajoutée, le dispositif de remplissage est fermé, et le cisaillement poursuivi de telle sorte que la vitesse de cisaillement se situe dans la gamme entre 10 000 et 40 000 s⁻¹, et
- ensuite, tout en maintenant les conditions de dispersion, un composé d'ammonium quaternaire basique, et éventuellement de l'eau avant l'addition du composé d'ammonium, sont ajoutés.
